# EUROPEAN PATENT APPLICATION

(11) **EP 4 599 826 A1**
(43) Date of publication of application: **13.08.2025**
(21) Application number: 24156047.3
(22) Date of filing: 06.02.2024
(51) Int. Cl.: A61K 31/198, A61K 45/06, A61P 35/00

(54) **LEUCINE FOR USE IN THE TREATMENT OF CANCER**

(71) Applicant: IFOM - Istituto Fondazione di Oncologia Molecolare ETS, 20139 Milano (IT)
(72) Inventor: Vernieri, Claudio, 20139 Milano (MI) (IT); Salvadori, Giulia, 20139 Milano (MI) (IT)
(74) Representative: Pace Napoleone, Maria

(57) **Abstract**

The invention relates to leucine as food supplement in the treatment of cancer. In particular leucine, when used as food supplement, enhances the effects of cycling fasting, such as the effects of a Fasting Mimicking Diet (FMD), and/or immune checkpoint inhibitors (ICIs), chemotherapy, chemotherapy plus immune checkpoint inhibitors, alone or in combination with fasting/FMD.

## Description

### FIELD OF THE INVENTION

The present invention relates to leucine as food supplement in the treatment of cancer. In particular leucine, when used as food supplement, enhances the effects of cycling fasting, such as the effects of a Fasting Mimicking Diet (FMD), and/or immune checkpoint inhibitors (ICIs), chemotherapy, chemotherapy plus immune checkpoint inhibitors, alone or in combination with fasting/FMD.

### BACKGROUND OF THE INVENTION

Breast cancer and lung cancer are the main leading cause of cancer death in women (17%) and men (27%) worldwide, respectively¹. In both sexes, colorectal cancer is the second leading cause of cancer death (11% among men and 12% among women). These tumors are associated with low 5-year survival rates when diagnosed in the metastatic stage¹. For this reason, lung, breast and colorectal cancer are considered the "three big killers" among human neoplasms, and are therefore the subject of many pre-clinical and clinical studies aimed at identifying new, effective, safe and economically sustainable therapies to prolong patient survival and, potentially, to increase tumor cure rates. In fact, despite recent therapeutic advances, which include new combinations of chemotherapy drugs, or the combination of chemotherapy with immunotherapy, these tumors are still associated with relatively high post-surgical recurrence rates and poor long-term outcomes¹. This highlights the necessity to find new strategies to improve the efficacy of chemo-immunotherapies regimens in patients with these tumor types.

Recent preclinical studies have shown that specific experimental nutritional interventions, such as cyclic fasting or fasting-mimicking diets (FMD), can produce additive or synergistic anticancer effects when combined with standard chemotherapy or immunotherapy in several syngeneic mouse models, including triple negative breast cancer, colorectal and lung cancer models ²⁻⁷. These data are consistent with the observation that fasting and FMD produce desirable immunomodulatory modifications, including an increase of tumor-infiltrating cytotoxic CD8+ T cells and a reduction of immunosuppressive regulatory T cells (Tregs) in syngeneic TNBC and lung mouse cancer models^{2,4}.

A recent clinical study conducted by the research group of present inventors in 101 cancer patients treated with standard therapies in combination with a five-day cyclic FMD regimen has shown that cyclic FMD is associated with positive immunomodulatory effects, leading to the downregulation of immunosuppressive myeloid-derived suppressor cells (MDSCs) and regulatory T cells (Tregs) at both systemic and tumor levels, paralleled by a boost of the antitumor immune compartment, including an increase in cytotoxic T lymphocytes, natural killer (NK) cells and T lymphocytes with memory phenotype⁸. In addition, some patients with metastatic TNBC, CRC or lung cancer enrolled in this trial, and undergoing cyclic FMD in combination with standard chemotherapy or immunotherapy, achieved complete and long-lasting tumor remissions⁹.

Based on these preclinical and clinical results, cyclic FMD is emerging as a promising antineoplastic nutritional intervention that could boost the anticancer activity of conventional anticancer treatments. Understanding the molecular mechanisms underlying FMD-induced immunomodulatory effects will help in the development of new strategies to enhance the immunomodulatory and, potentially, antitumor effects of cyclic FMD, either alone or in combination with immune checkpoint inhibitors (ICIs), chemotherapy, or a combination of chemotherapy and ICIs.

### SUMMARY OF THE INVENTION

In the present invention, the inventors demonstrated that cyclic fasting in mice is associated with specific metabolic changes, including the modulation of systemic and intratumor metabolism of branched chain amino acids (BCAAs), namely leucine, isoleucine and valine (BCAA). Starting from this finding, they investigated whether fasting/FMD-induced increase of blood BCAA levels could contribute to the immunomodulatory and antitumor effects of nutrient starvation. For instance, leucine is a potent activator of mTORC1^{10,11}, which plays a crucial role in T lymphocyte activation and proliferation in response to antigen exposure. The present invention demonstrates that oral leucine supplementation delays tumor progression and improves the survival of tumor-bearing mice. In addition, oral leucine supplementaton significantly potentiates fasting/FMD-induced immunomodulatory and anticancer effects, and this synergistic antitumor effects could derive from the cooperation of oral leucine supplementation and fasting/FMD in increasing blood leucine concentration. Finally, oral leucine supplementation, both alone and in combination with fasting/FMD, potentiates the efficacy of ICIs, chemotherapy, or combinations of chemotherapy and ICIs in the treatment of these highly aggressive tumor types.

Therefore the combination of leucine supplementation with fasting/FMD and/or with an optimized chemotherapy, immunotherapy or chemoimmunotherapy treatment is particularly advantageous in that it enhances the anti-cancer effect of cyclic fasting or FMD, either alone or in combination with chemotherapy, immunotherapy or chemoimmunotherapy. Specifically, the combination of the invention is able to delay tumor progression given that leucine, when used as food supplement, potentiates the efficacy of further therapeutic interventions, such as chemotherapy, immunotherapy or chemoimmunotherapy combinations, and also leads to increased cancer cure rates in some models of breast cancer and colorectal cancer. The combination of the invention is particularly advantageous also because it is safe and very well tolerated, with no toxicities emerging when oral leucine is combined with fasting, chemotherapy, chemotherapy plus fasting, immunotherapy, immunotherapy plus fasting, chemoimmunotherapy, chemoimmunotherapy plus fasting. The object of the present invention is the use of oral leucine supplementation as an umbrella anticancer therapy that could be implemented in several clinical contexts in patients with different tumor types and receiving different standard-of-care concomitant therapies.

The various embodiments of the invention are presented in the detailed description below and the preferred embodiment are presented in the claims which form an integral part of the present description.

### DETAILED DESCRIPTION OF THE INVENTION

It is an object of the invention leucine or a pharmaceutically acceptable salt thereof for use in the treatment of cancer, either used alone or in combination with at least one fasting cycle or with a fasting mimicking diet (FMD) plus/minus further therapeutic intervention; preferably said at least one fasting cycle is characterized by 24 h to 72 h, preferably 24 h to 48 h, more preferably 48 h of water-only fasting (100% caloric restriction with unlimited access to water); still preferably said fasting mimicking diet lasts for a period of 24 to 190 hours, preferably said fasting mimicking diet lasts for a period of 24 to 150 hours, preferably said fasting mimicking diet lasts for approximately 120 hours (5 days).

In a further preferred embodiment of the invention said fasting mimicking diet is a regular caloric intake reduced by 65% to 85% and/or said fasting mimicking diet is based on an average caloric intake of between 300-600 Kcal/day.

Preferably the fasting mimicking diet comprises a first period of 0 to 24 hours wherein the caloric intake is a regular caloric intake reduced by approximately 65%, followed by a second period of 24 to 96 hours wherein the caloric intake is a regular caloric intake reduced by approximately 85%, preferably the first period lasts approximately 24 hours and the second period lasts approximately between 48 and 96 hours.

In a further preferred embodiment of the invention said fasting mimicking diet comprises a reduced protein intake and/or a reduced simple carbohydrate (sugars) intake, and/or a relatively increased content of unsaturated fat intake.

Still preferably said fasting mimicking diet comprises administering a food having a relative content of monounsaturated and/or polyunsaturated fats between 10 and 80 %, a content of proteins from 0 to 10 % and a content of carbohydrates from 5 to 50 %.

It is a further object of the invention leucine or the pharmaceutically acceptable salt thereof for use as defined above and/or in combination with a further therapeutic intervention selected from the group consisting of: surgery, radiotherapy and a further therapeutic agent.

Preferably said further therapeutic agent is an immune checkpoint inhibitor (ICI), preferably a PD-1 inhibitor, and/or PDL-1 inhibitor and/or CTLA-4 inhibitor.

Still preferably said further therapeutic agent is a chemotherapeutic agent, preferably said chemotherapeutic agent is selected from the group consisting of: a DNA synthesis inhibitor, a DNA damaging agent, a topoisomerase inhibitor, a microtubule poison, preferably said chemotherapeutic agent is selected from the group consisting of: oxaliplatin, carboplatin, cisplatin, paclitaxel, docetaxel, nab-paclitaxel, eribulin, doxorubicin, epirubicin, irinotecan, gemcitabine, cyclophosphamide.

It is a further object of the invention leucine or the pharmaceutically acceptable salt thereof for use as defined above wherein said cancer is resistant to conventional therapy, preferably said cancer is resistant to radiotherapy or chemotherapy, preferably said cancer is resistant to: a DNA synthesis inhibitor, a DNA damaging agent, a topoisomerase inhibitor, a microtubule poison, preferably said chemotherapeutic agent is selected from the group consisting of: oxaliplatin, carboplatin, cisplatin, paclitaxel, docetaxel, nab-paclitaxel, eribulin, doxorubicin, epirubicin, irinotecan, gemcitabine, cyclophosphamide.

Preferably said cancer is selected from the group consisting of: breast cancer, colorectal cancer, small cell and non small cell lung carcinomas (SCLCs and NSCLCs), pancreatic adenocarcinoma, colon cancer, rectal cancer, mucinous adenocarcinoma, melanoma, biliary tract carcinoma, gastric carcinoma, esophageal carcinoma, small intestine carcinoma, neuroblastomas, gliomas, sarcomas, lymphomas, leukemias, neuroendocrine carcinomas, preferably said cancer is either primary and metastatic cancer.

It is a further object of the invention leucine or the pharmaceutically acceptable salt thereof for use as defined above wherein leucine is administered in an amount of approximately between 100 mg/kg and 1000 mg/kg on a daily basis, preferably between 150 mg/kg and 800 mg/kg on a daily basis, preferably between 250 mg/kg and 600 mg/kg on a daily basis.

It is a further object of the invention paharmaceutical or nutritional composition or dietary supplement comprising leucine or the pharmaceutically acceptable salt thereof for use as defined above, and a pharmaceutical or nutritionally acceptable carrier.

Preferably the pharmaceutical or nutritional composition or dietary supplement is formulated as an oral formulation with delayed or prolonged intestinal absorption.

The present invention provides leucine and derivatives thereof or a pharmaceutically acceptable salt of leucine or its derivatives, for use as a food supplement in a method for treating cancer. Leucine may be in racemic form, which means that the compound comprises about equal amounts of enantiomers. Alternatively it may be present in an enantiomeric excess of either the L-enantiomer or the D-enantiomer. In one embodiment, leucine is present in an enantiomeric excess of the L-enantiomer. The racemic and enantiomeric forms may be obtained in accordance with known procedures in the art. Leucine derivatives include carboxylgroup derivatives and amino-group derivatives. Carboxyl group derivatives include L-leucine methyl ester (H-Leu-OMe), L-leucine ethyl ester (H-Leu-OEt), L-leucine allyl ester (H-Leu-OAll), L-leucine 2-propoxyethyl ester (H-Leu-O(CH₂)₂OPr), L-leucine t-butyl ester (H-Leu-OtBu), L-leucine benzyl ester (H-Leu-OBzl), l-leucinylmethoxymethane (H-Leu-OCH₂OMe), L-leucine hydroxamate (H-Leu-NHOH). Amino group derivatives include N-acetyl-L-leucine (Ac-Leu-OH), N-formyl-L-leucine (For-Leu-OH), N-Methyl-L-leucine (Me-Leu-OH).

A "pharmaceutically acceptable salt" as referred to herein, is any salt preparation that is appropriate for use in a pharmaceutical application or in a food supplement. Pharmaceutically acceptable salts include, but are not limited to, amine salts, such as N,N'-dibenzylethylenediamine, chloroprocaine, choline, ammonia, diethanolamine and other hydroxyalkylamines, ethylenediamine, N-methylglucamine, procaine, N-benzylphenethylamine, l-para- chloro- benzyl-2-pyrrolidin-i'-ylmethylbenzimidazole, diethylamine and other alkylamines, piperazine, tris(hydroxymethyl)aminomethane and the like; alkali metal salts, such as lithium, potassium, sodium and the like; alkali earth metal salts, such as barium, calcium, magnesium and the like; transition metal salts, such as zinc, aluminum and the like; other metal salts, such as sodium hydrogen phosphate, disodium phosphate and the like; mineral acids, such as hydrochlorides, sulfates and the like; and salts of organic acids, such as acetates, lactates, malates, tartrates, citrates, ascorbates, succinates, butyrates, valerates, fumarates and the like.

A "pharmaceutically or nutritionally acceptable carrier" as referred to herein, is any known compound or combination of known compounds that are known to those skilled in the art to be useful in formulating pharmaceutical compositions or nutritional compositions such as food supplements. In a further embodiment, the pharmaceutically or nutritionally acceptable carrier may be a solid, and the composition may be in the form of a powder or tablet. A solid pharmaceutically or nutritionally acceptable carrier may include, but is not limited to, one or more substances which may also act as flavouring agents, buffers, lubricants, stabilisers, solubilisers, suspending agents, wetting agents, emulsifiers, dyes, fillers, glidants, compression aids, inert binders, sweeteners, preservatives, dyes, coatings, or tablet-disintegrating agents. The carrier may also be an encapsulating material. In powders, the carrier is a finely divided solid that is in admixture with the finely divided active agents according to the present disclosure. In tablets, the active agent may be mixed with a carrier having the necessary compression properties in suitable proportions and compacted in the shape and size desired. The powders and tablets, for example, contain up to 99% of the active agents. Suitable solid carriers include, for example, calcium phosphate, magnesium stearate, talc, sugars, lactose, dextrin, starch, gelatin, cellulose, polyvinylpyrrolidine, low melting waxes and ion exchange resins. In another embodiment, the pharmaceutically or nutritionally acceptable carrier maybe a gel and the composition maybe in the form of a cream or the like. In yet a further embodiment, the carrier may include, but is not limited to, one or more excipients or diluents. Examples of such excipients are gelatin, gum arabicum, lactose, microcrystalline cellulose, starch, sodium starch glycolate, calcium hydrogen phosphate, magnesium stearate, talcum, colloidal silicon dioxide and the like.

The pharmaceutical or nutritional composition or dietary supplement can also be formulated as an oral formulation with delayed or prolonged intestinal absorption. As formulation with delayed or prolonged intestinal absorption it is intended that the composition is formulated in a way allowing a release into the body of a specified amount over a specified period of time of an active ingredient, namely a specific pharmacokinetic profile. The above expression is equivalent to controlled release and encompasses all the type of releases that are modified in comparison to an immediate release. This type of release means that the release is prolonged over time in comparison to an immediate release, i.e. it means that the active ingredient is released slowly over time, allowing a less frequent intake of the composition for the patient, or simply an optimized absorption of leucine to achieve higher and more durable therapeutic concentrations of leucine in the blood and in the tumor microenvironment.

In another embodiment, the pharmaceutically or nutritionally acceptable carrier may be a liquid, and the pharmaceutical composition or the food supplement is in the form of a solution. Liquid carriers are used in preparing solutions, suspensions, emulsions, syrups, elixirs and pressurized compositions. The active agent according to the present disclosure may be dissolved or suspended in a liquid carrier such as water, an organic solvent, a mixture of both or pharmaceutically acceptable oils or fats. The liquid carrier can contain other suitable pharmaceutical additives such as solubilisers, emulsifiers, buffers, preservatives, sweeteners, flavouring agents, suspending agents, thickening agents, colours, viscosity regulators, stabilizers or osmo-regulators. Suitable examples of liquid carriers for oral and parenteral administration include water (partially containing additives as above, e.g. cellulose derivatives, such as sodium carboxymethyl cellulose solution), alcohols (including monohydric alcohols and polyhydric alcohols, e.g. glycols) and their derivatives, and oils (e.g. fractionated coconut oil and arachis oil). For parenteral administration, the carrier can also be an oily ester such as ethyl oleate and isopropyl myristate. Sterile liquid carriers are useful in sterile liquid form compositions for parenteral administration. The liquid carrier for pressurised compositions can be a halogenated hydrocarbon or other pharmaceutically acceptable propellant.

Liquid pharmaceutical compositions, which are sterile solutions or suspensions, can be administered through, for example, intramuscular, intrathecal, epidural, intraperitoneal, intravenous and subcutaneous injection. The active agent may be prepared as a sterile solid composition that may be dissolved or suspended at the time of administration using sterile water, saline, or other appropriate sterile injectable medium.

The agents and compositions of the present disclosure may be administered orally in the form of a sterile solution or suspension containing other solutes or suspending agents (for example, enough saline or glucose to make the solution isotonic), bile salts, acacia, gelatin, sorbitan monoleate, polysorbate 80 (oleate esters of sorbitol and its anhydrides copolymerized with ethylene oxide) and the like. The agents used according to the disclosure can also be administered orally either in liquid or solid composition form. Compositions suitable for oral administration include solid forms, such as pills, capsules, granules, tablets, and powders, and liquid forms, such as solutions, syrups, elixirs, and suspensions. Forms useful for parenteral administration include sterile solutions, emulsions, and suspensions.

Leucine, including its salts and derivatives, and compositions comprising the same may alternatively be administered by inhalation (e.g. intranasally). Compositions may also be formulated for topical use. For instance, creams or ointments may be applied to the skin.

In one embodiment, the pharmaceutical composition or the nutritional composition is in the form of a tablet. In tablets, the active agent may be mixed with a vehicle having the necessary compression properties in suitable proportions and compacted in the shape and size desired. The tablets may contain up to 99% by weight of leucine or a salt thereof or a derivative thereof.

Thus, in one embodiment, leucine, or the pharmaceutically acceptable salt of leucine, is provided in a solid dosage form suitable for oral administration, notably in the form of a tablet.

The above-mentioned pharmaceutical or nutritional formulations in solid oral dosage form, such as tablets, may be prepared by any method known in the art of pharmacy. Said formulations are usually prepared by mixing the active substance, or a pharmaceutically acceptable salt thereof, with conventional pharmaceutically acceptable carriers, diluents or excipients.

Leucine, or a pharmaceutically acceptable salt of the same, may be administered at a daily dose between 100 mg/kg and 1000 mg/kg, preferably between 150 mg/kg and 800 mg/kg (for example between 250 mg/kg and 600 mg/kg etc etc), optionally by solid oral or liquid oral route.

The total daily dose may be divided in multiple administrations, e.g. one administration may be required two or more times a day to achieve the required dose. As an example, the required number of tablets to provide the total daily dose of leucine may be split into two daily administrations (for example, in the morning and evening) or three administrations (for example, in the morning, noon and evening).

Treatment duration may vary according to tumor response, duration of tumor response, relapse-free survival (RFS), disease-free survival (DFS) and Event-free survival (EFS) in patients with surgically resected tumor, progression-free survival (PFS) and overall survival (OS) in patients with advanced malignancies, or other clinical factors, including treatment tolerability and quality of life. It may be seven days or more, two weeks or more, three weeks or more, one month or more, six weeks or more, seven weeks or more or two months or more. For example, it is three months or more, four months or more, five months or more or even six months or more.

Any and all combinations of dosage form, dose amount, dosing schedule and treatment duration are envisaged and encompassed by the disclosure. An example combination is a total daily dose between 4.5 g and 10 g per day, taken across three administrations per day, for a treatment duration of two months or more. A further example combination is a total daily dose of more than 4 g to no more than 5 g per day, taken across three administrations per day, for a treatment duration of six months or more. The dosage form may be, for example, a solid oral dosage form, notably tablets. A "subject", as used herein, may be a vertebrate, mammal or domestic animal. Hence, compositions according to the disclosure may be used to treat any mammal, for example livestock (e.g. a horse), pets, or may be used in other veterinary applications. Further for example, the subject is a human. In the present invention leucine is used as food supplement for treatment of cancer in combination with a specific caloric intake regime. Said specific caloric intake is based on a reduced daily caloric intake compared to a regular daily caloric intake, in particular it involves a specific daily caloric intake and a specific macronutrient intake as defined below.

In the present invention, a specific caloric and macronutrient intake may be achieved, for example, by means of fasting or of a fasting mimicking diet (FMD).

Fasting, or a fasting cycle according to the invention, involves about 2 days of nutrient starvation, with free consumption to water. Therefore, said fasting cycle is 100% reduced caloric intake.

"Fasting mimicking diet" (FMD) refers to previously described formulations to mimic the effects of fasting.⁸ Complete fasting results to be challenging for cancer patiens, especially when undergoing chemotherapy, so the inventors have developed a FMD that enables a patient to eat "food" while achieving the same effects of fasting on normal and cancer cells.

The fasting or FMD is started one or two days before the administration of pharmacological therapies (e.g., chemotherapy, immunotherapy, chemoimmunotherapy, taegeted therapies) and continues for the following 2-7 days, preferably for the following 2-4 days while the therapy is most active.

In a preferred embodiment, FMD comprises one or more FMD cycles, each cycle consisting of 1-7 days (preferably of 2-5 days, more preferably 5 days) of low-calorie intake, for example as follows:

### Day 1:

Mouse = approximately 50% of regular caloric intake
Human = approximately 35% of regular caloric intake (i.e. regular caloric intake reduced by approximately 65%)

### Days 2-5:

Mouse = approximately 10% of regular caloric intake (i.e. regular caloric intake reduced by approximately 90%)
Human = approximately 15% of regular caloric intake (i.e. regular caloric intake reduced by approximately 85%).

A further example of FMD cycles in human is as follows:
Dav 1:
   Human = approximately 50% of regular caloric intake
Days 2-5:
   Human = approximately 30% of regular caloric intake (i.e. regular calorie intake reduced by approximately 65%).

Other known protocols of FMD can be used within the invention. For example a further preferred reduced caloric intake is as follows:
Day 1: 54% caloric intake, about 1,090 kcal (10% protein, 56% fat, 34% carbohydrate)
Days 2-7: 20-34% caloric intake, about 426-725 kcal (5.3-9% protein, 26-44% fat, 27.6-47% carbohydrate).

In the present invention preferably the reduced caloric intake is obtained by fasting or by means of dietetic food with reduced caloric and/or protein content and/or carbohydrate content but containing all necessary micro nutrients to prevent malnutrition.

FMD is achieved with a low protein and low sugar and relatively high fat plant-based formulation followed by a standard/regular ad libitum diet until the complete recovery of bodyweight.

The reduction is compared to a regular caloric intake per day. A regular caloric intake for an adult, calculated on average for male and female, is of about 1800 Kcal/day. As used herein, regular intake is intended as about 1800 Kcal/day.

In the present invention the period of reduced caloric intake is repeated one or more times after respective periods of 3-60 days of regular caloric intake. According to the invention, Leucine supplementation is given to the subject both during the period(s) of reduced caloric intake and during the period(s) of regular caloric intake.

In a preferred embodiment said mammal is fed with a food having a relative content of monounsaturated and/or polyunsaturated fats between 10 and 80 %, a content of proteins from 0 to 10 % and a content of carbohydrates from 5 to 50 %.

Examples of useful fasting mimicking diets in the context of the present invention are set forth in U. S. Pat. Appl. Nos. 14/273,946 filed May 9, 2014; 14/497,752 filed September 26, 2014; 12/910,508 filed October 22, 2010; 13/643,673 filed October 26, 2012; 13/982,307 filed July 29, 2013; 14/060,494 filed October 22, 2013; 14/178,953 filed February 12, 2014; 14/320,996 filed July 1, 2014; 14/671,622 filed March 27, 2015. Additionally informative examples of FMD diets are also found in US patent application Ser. No. 15/148,251 and WIPO Pub. No. WO2011/050302.; WO2020/261131.

Usually, humans undergoing one or more FMD cycles do not lose more than 10% of body weight. FMD cycles are feasible and safe thus individuals well tolerate the diet. Reasons for stopping the FMD are not related to heathy status but usually to non-compliance to the dietary protocol or for work scheduling issues. However, there are clinical condition which can make the individual not eligible to FMD, such as being underweight, or being unable to at least partially restore the weight that was lost during the FMD.

The present invention will be illustrated by means of non-limiting examples and figures as follows.

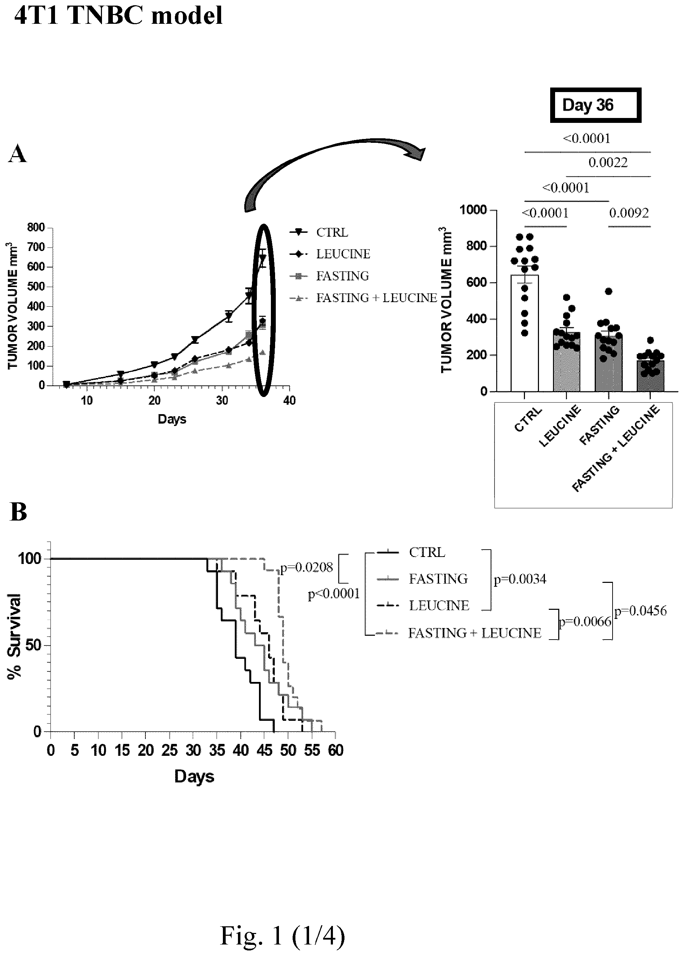

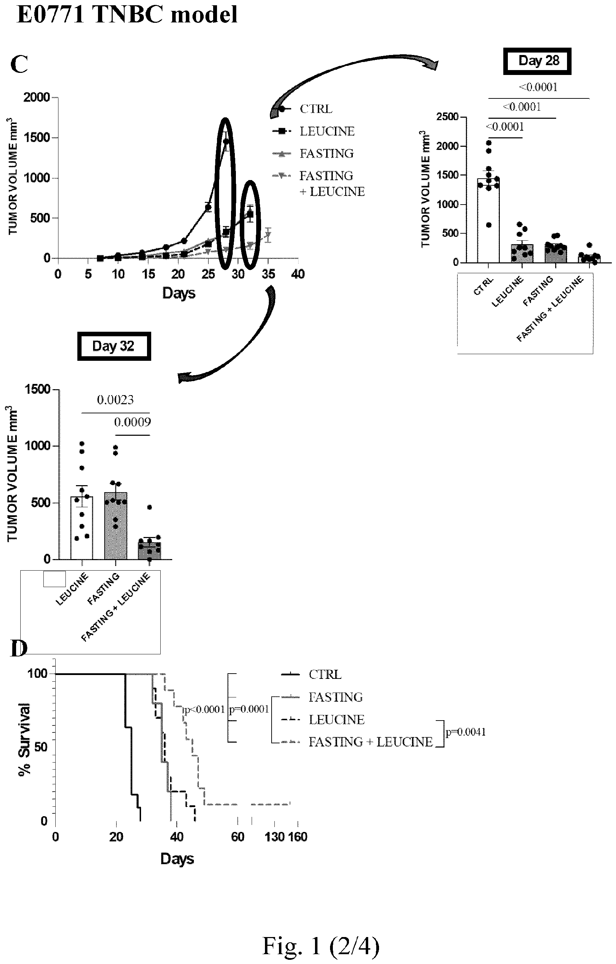

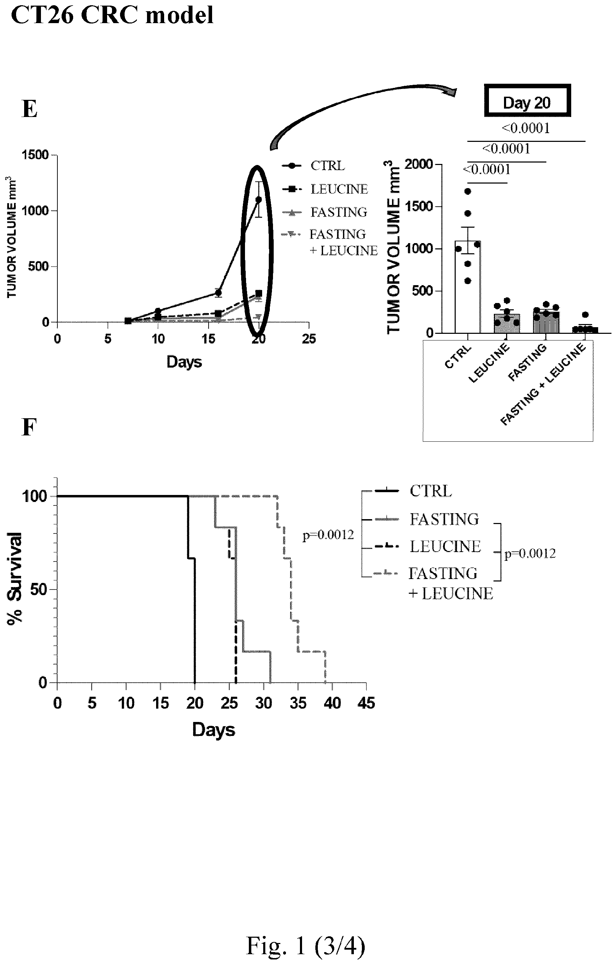

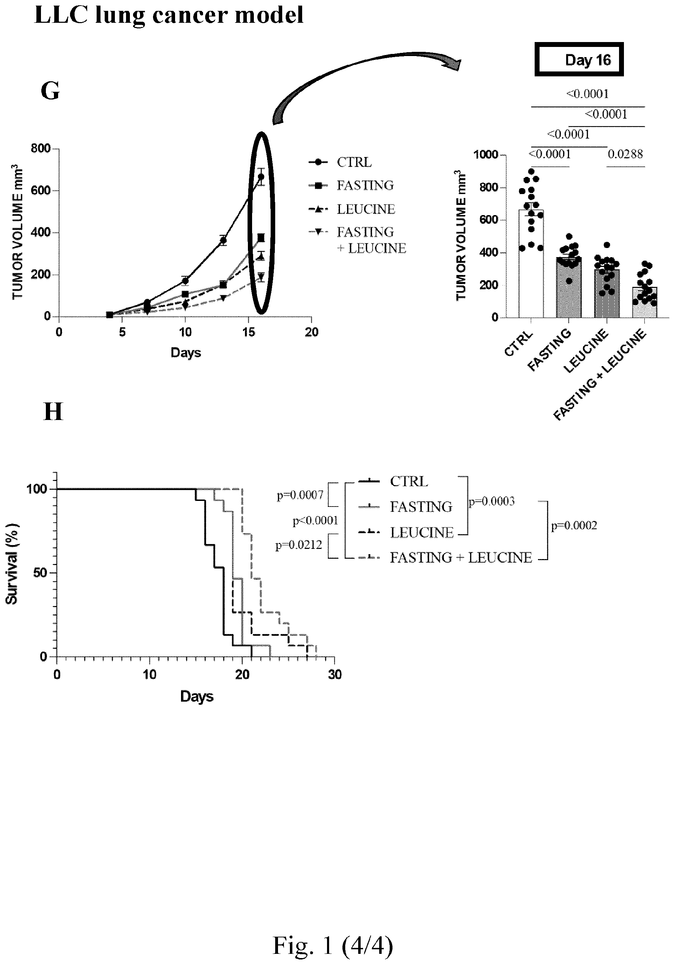

**FIG 1****. Leucine, alone or combined with 48h fasting, delays cancer progression and significantly prolongs mouse survival in 4 different syngeneic models.**

A) Growth of 4T1-luc (constitutively expressing luciferase) cell transplants in the mammary fat pad of 7-weeks old female BALB/c mice fed with standard diet or fasting cycles (48-hours of water-only fasting, followed by *ad libitum* refeeding, and repeated every 7 days), alone or combined with Leucine supplementation (500 mg/kg) administered once a day, as given through oral gavage (n=14). B) Survival curves of mice treated as described in A) are reported. C) Growth of E0771 cell transplants after cell injection in the mammary fat pad of 7-weeks old female C57BL6/J mice fed with standard diet or fasting cycles, alone or combined with oral Leucine (500 mg/kg) administered once a day (n=9-10). D) Survival curves of mice treated as described in C) are reported. E) Growth of CT26 CRC cell transplants after cell injection in the right flank of 7-weeks old female BALB/c mice fed with standard diet or fasting cycles, alone or combined with Leucine (500 mg/kg) administered once a day, by oral gavage (n=6). F) Survival curves of mice treated as described in E) are reported. G) Growth of LLC lung cell transplants after cell injection in the right flank (subcutaneous tissue) of 7-weeks old female C57BL6/J mice fed with standard diet or fasting cycles, alone or combined with leucine (500mg/kg) administered once a day, by oral gavage (n=5). H) Survival curves of mice treated as described in G) are reported.

Data are represented as mean ± SEM. P values were determined by ordinary one-way Anova. Comparison of survival curves were performed with Log-rank (Mantel-Cox) test. P values ≤ 0.05 were considered significant.

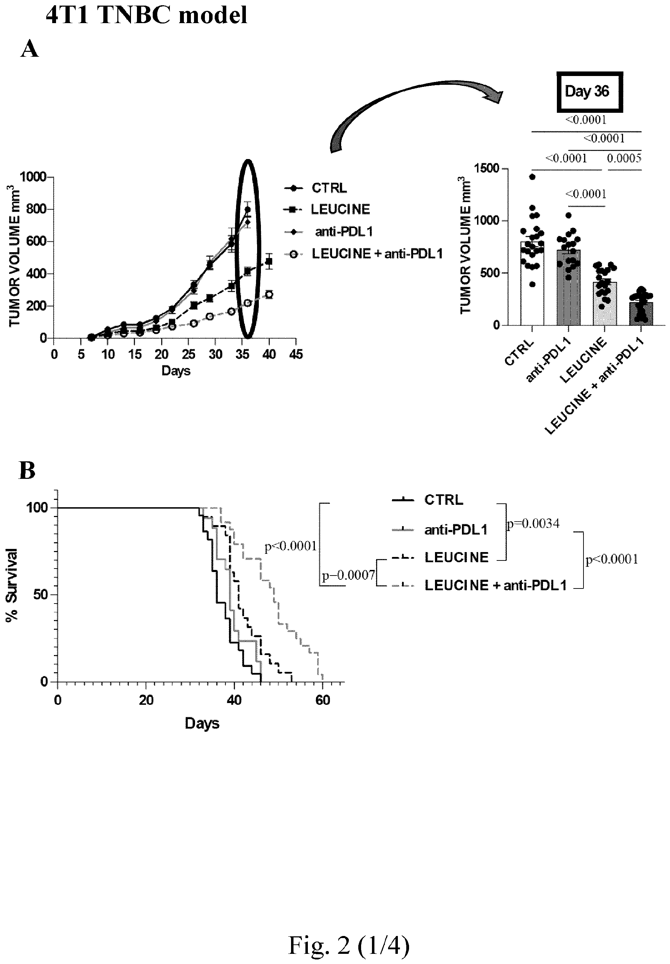

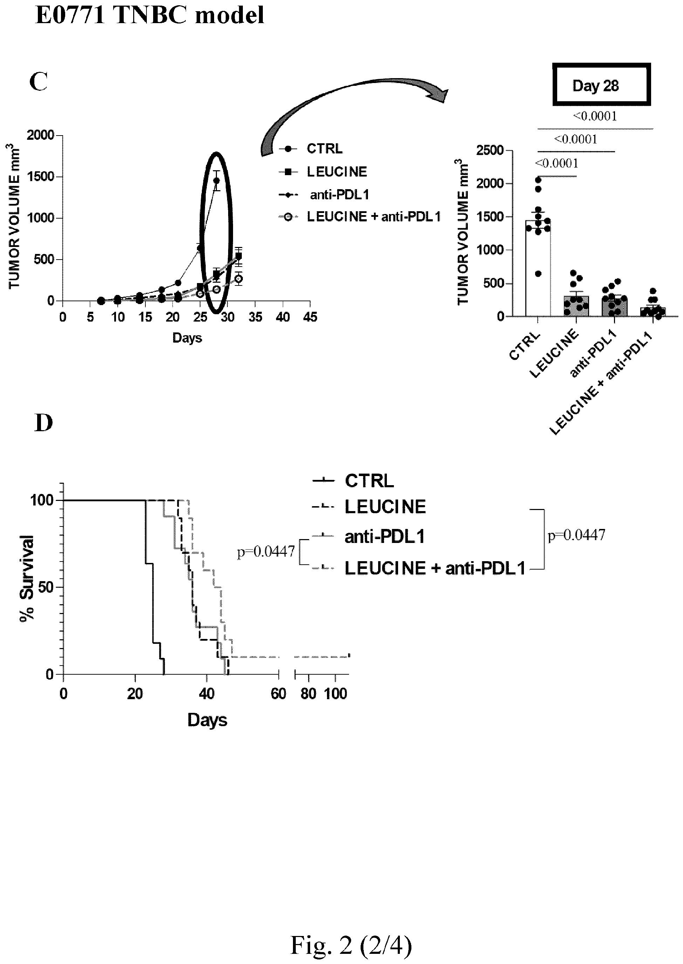

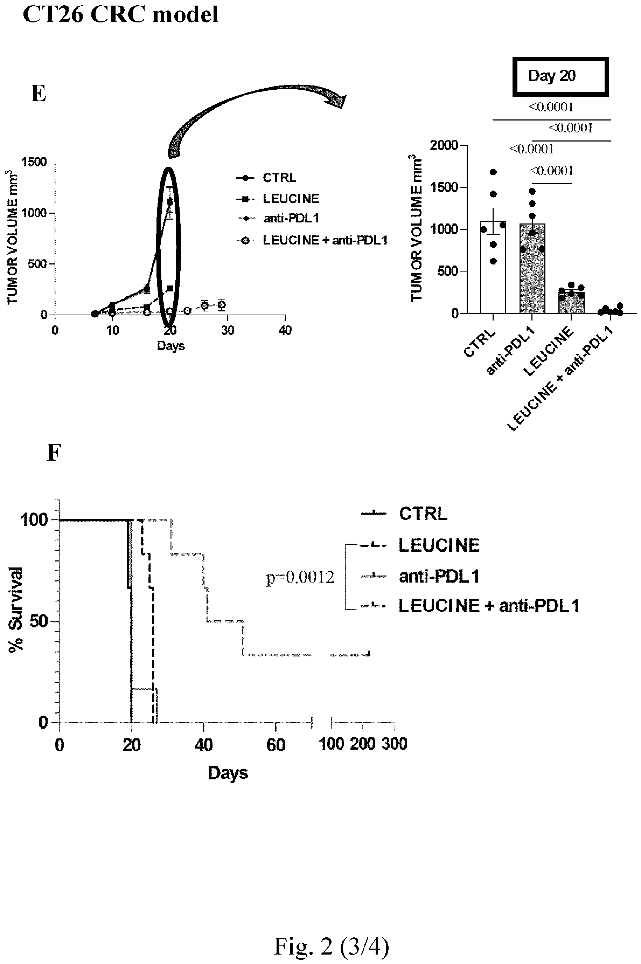

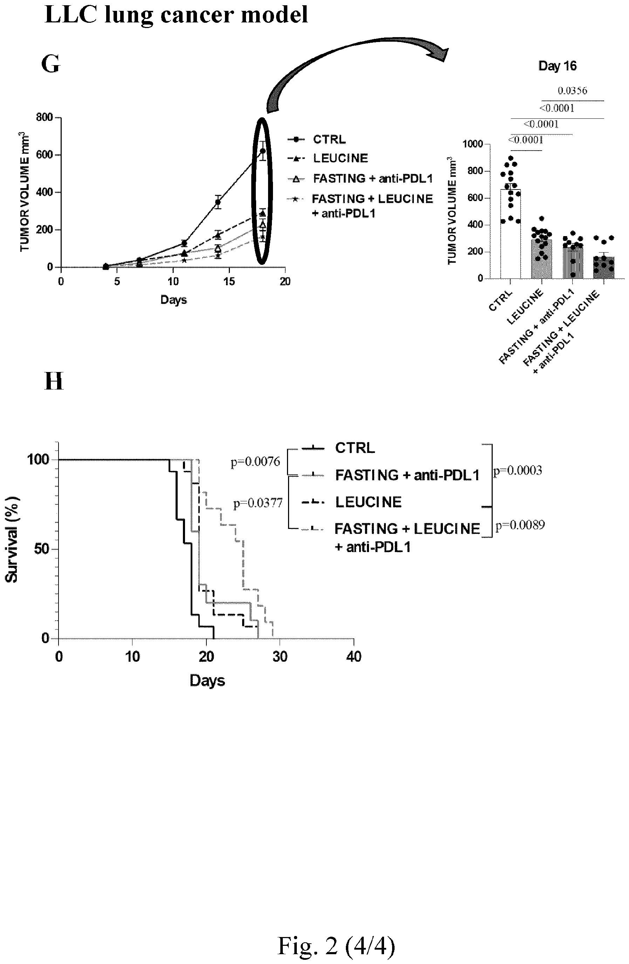

**FIG 2****. Impact of Leucine supplementation, alone or combined with anti-PD-L1 immunotherapy, on tumor growth and animal survival.**

A) Growth of 4T1-luc cell transplants after cell injection in the mammary fat pad of 7-weeks old female BALB/c mice treated with oral Leucine (500 mg/kg), as administered once a day by oral gavage, plus/minus murine anti-PDL1 Monoclonal Antibody (MoAb) (100ug in each mouse) every-other-day by intraperitoneal injection, for a total number of 4 anti-PD-L1 treatment cycles, (n=18-23). B) Survival curves of animals, as treated as described in A), are reported. C) Growth of E0771 cell transplants in the mammary fat pad of 7-weeks old female C57BL6/J mice treated with Leucine (500 mg/kg), as administered once a day by oral gavage, plus/minus anti-PDL1 MoAb (100ug per mouse) every other day, for a total of 4 cycles, by intraperitoneal injection (n=9-10). D) Survival curves of mice treated as described in C) are reported. E) Growth of CT26 CRC cell transplants in the right flank of 7-weeks old female BALB/c mice fed with standard diet or fasting cycles, alone or combined with Leucine (500 mg/kg), as administered once per day by oral gavage, plus/minus anti-PDL1 MoAb (100ug per mouse) every other day, for a total of 4 cycles, by intraperitoneal injection (n=6). F) Survival curves of animals treated as described in E) are reported. G) Growth of LLC lung cell transplants after cell injection in the right flank (subcutaneous tissue) of 7-weeks old female C57BL6/J fed with standard diet or fasting cycles, alone or combined with Leucine (500 mg/kg), as administered once per day by oral gavage, plus/minus anti-PDL1 MoAb (100ug per mouse) every other day, for a total of 4 cycles, by intraperitoneal injection (n=10-15). H) Survival curves of mice treated as described in G) are reported.

Data are represented as mean ± SEM. P values were determined by ordinary one-way Anova. Comparison of survival curves were performed with Log-rank (Mantel-Cox) test. P values ≤ 0.05 were considered significant.

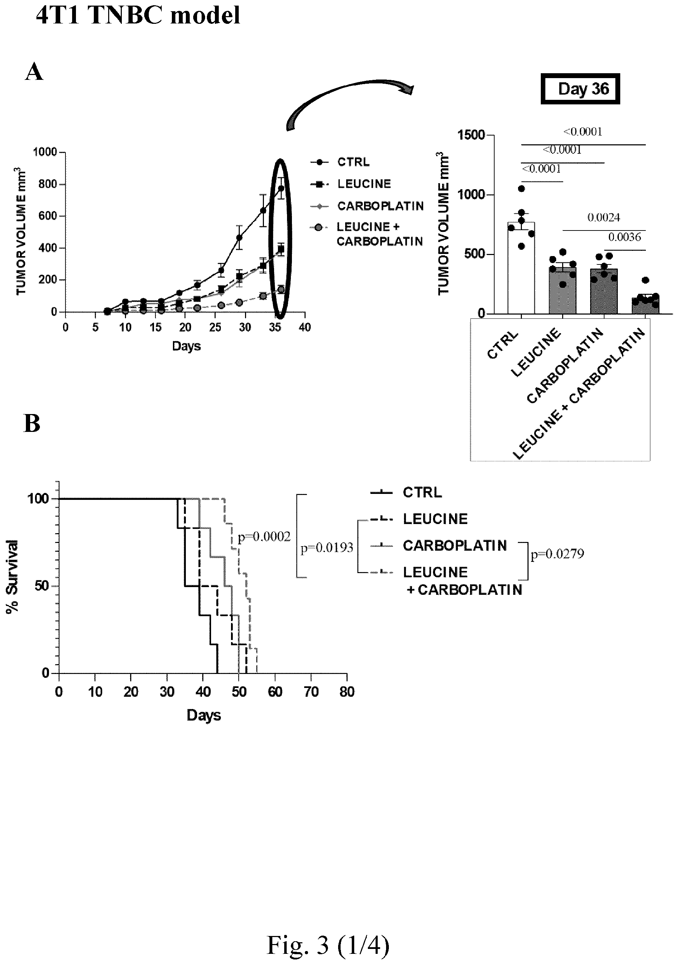

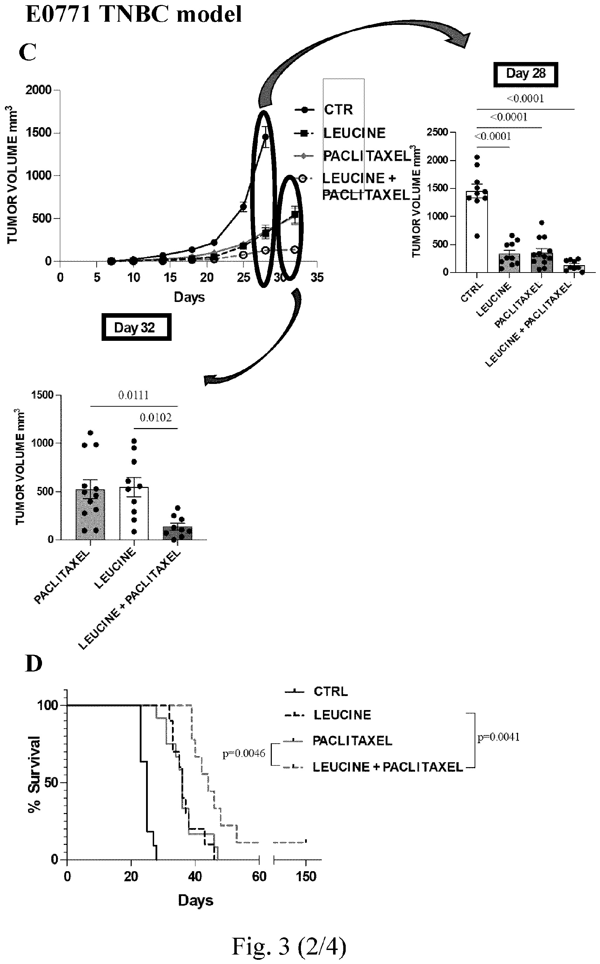

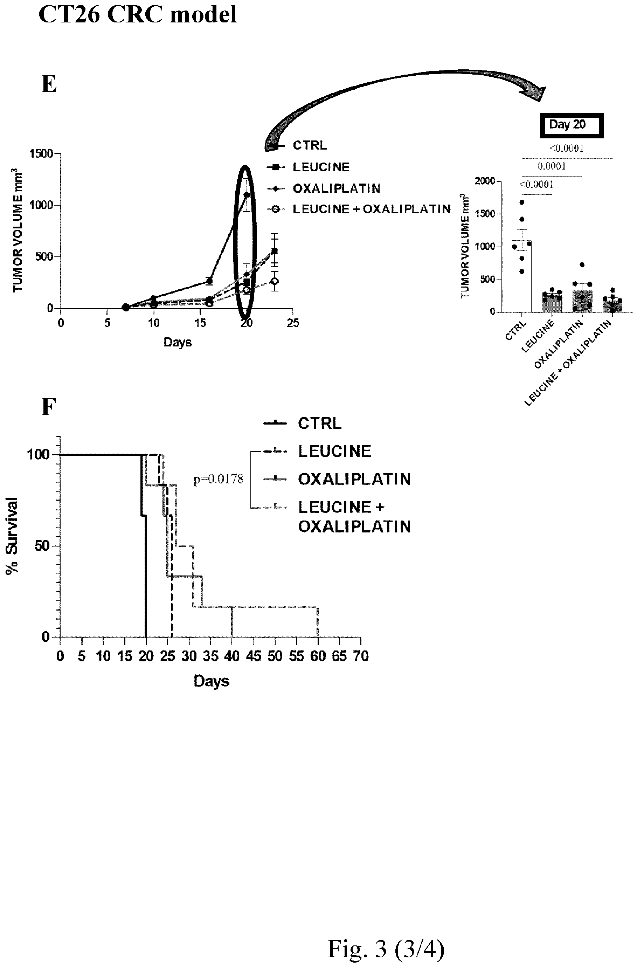

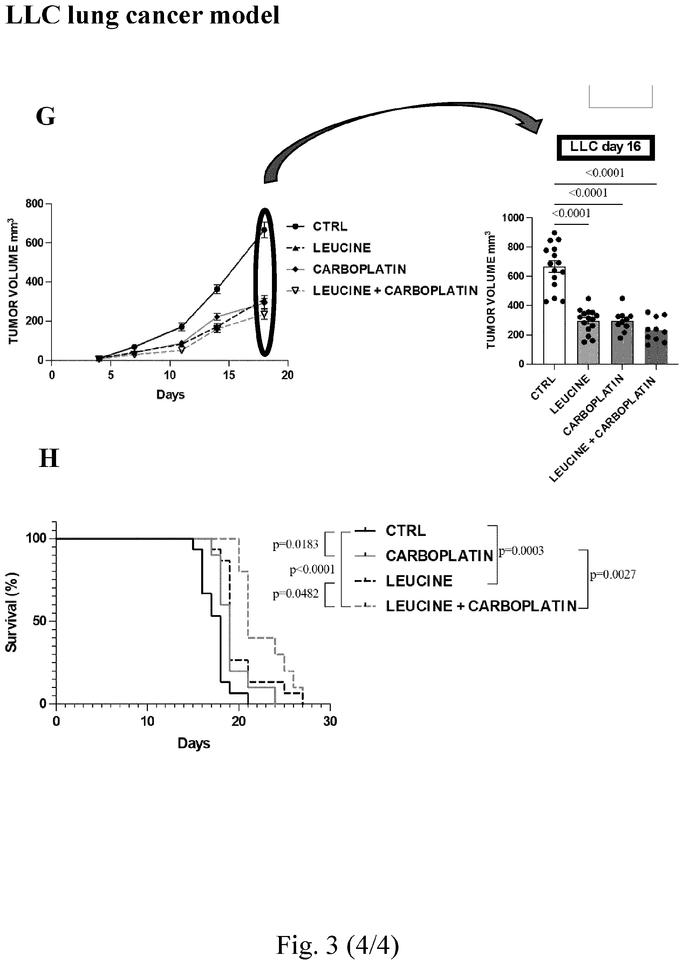

**FIG 3****. Impact of Leucine supplementation, alone or combined with platinum chemotherapy, on tumor growth and animal survival.**

A) Growth of 4T1-luc cell transplants in the mammary fat pad of 7-weeks old female BALB/c mice treated with Leucine (500 mg/kg), as administered once a day by oral gavage, plus/minus carboplatin (50 mg/kg), as administered once a week by intraperitoneal injection (n=6). B) Survival curves of animals treated as described in A) are reported. C) Growth of E0771 cell transplants in the mammary fat pad of 7-weeks old female C57BL6/J mice treated with Leucine (500 mg/kg), as administered once a day by oral gavage, plus/minus paclitaxel (30 mg/kg), as administered once a week by intraperitoneal injection (n=9-12). D) Survival curves of animals treated as described in C) are reported. E) Growth of CT26 cell transplants in the right flank of 7-weeks old female BALB/c mice treated with Leucine (500 mg/kg), as administered once a day by oral gavage, plus/minus oxaliplatin (5 mg/kg), as administered once a week by intraperitoneal injection (n=6). F) Survival curves of animals treated as described in E) are reported. G) Growth of LLC lung cell transplants after cell injection in the right flank (subcutaneous tissue) of 7-weeks old female C57BL6/J treated with Leucine (500 mg/kg), as administered once a day by oral gavage, plus/minus carboplatin (50 mg/kg), as administered once a week by intraperitoneal injection (n=10-15). H) Survival curves of mice treated as described in G) are reported.

Data are represented as mean ± SEM. P values were determined by ordinary one-way Anova. Comparison of survival curves were performed with Log-rank (Mantel-Cox) test. P values ≤ 0.05 were considered significant.

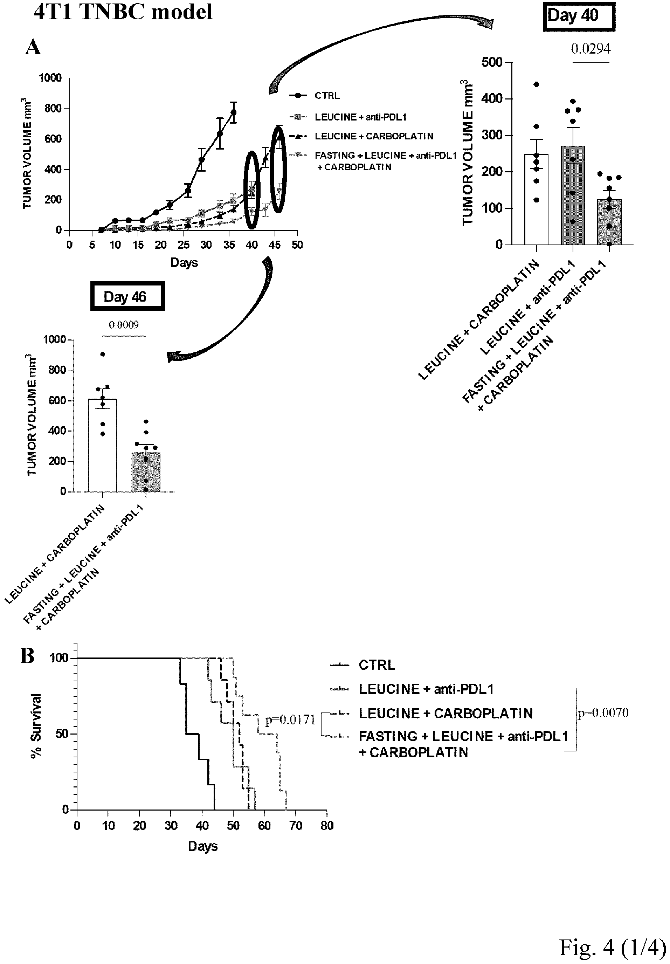

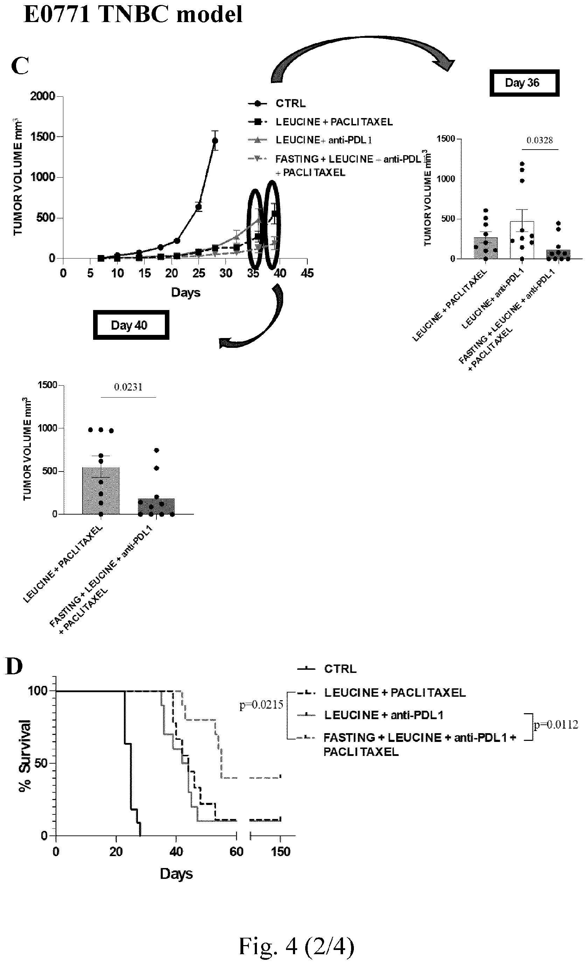

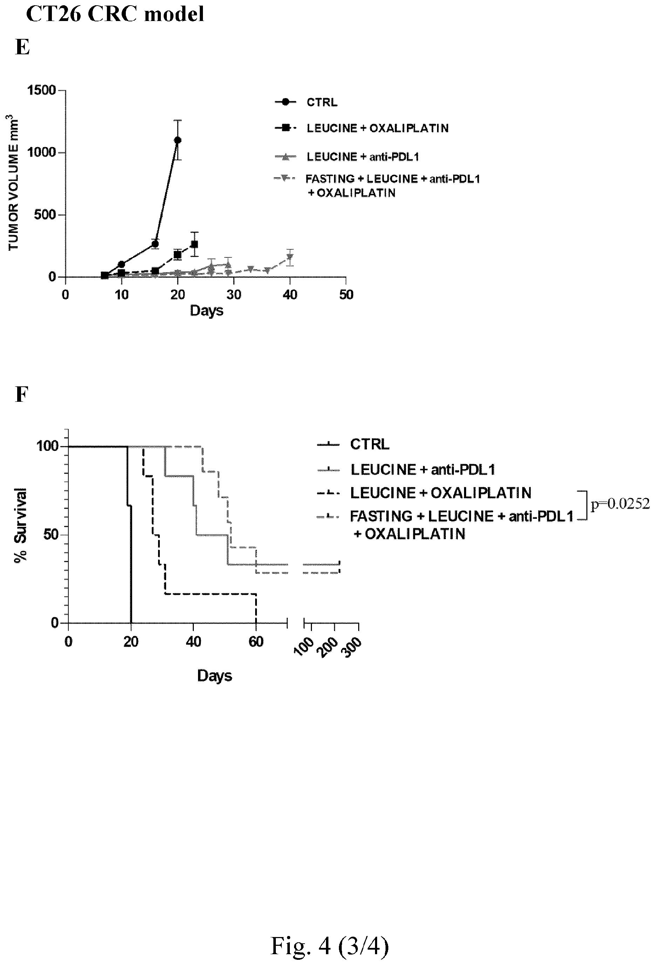

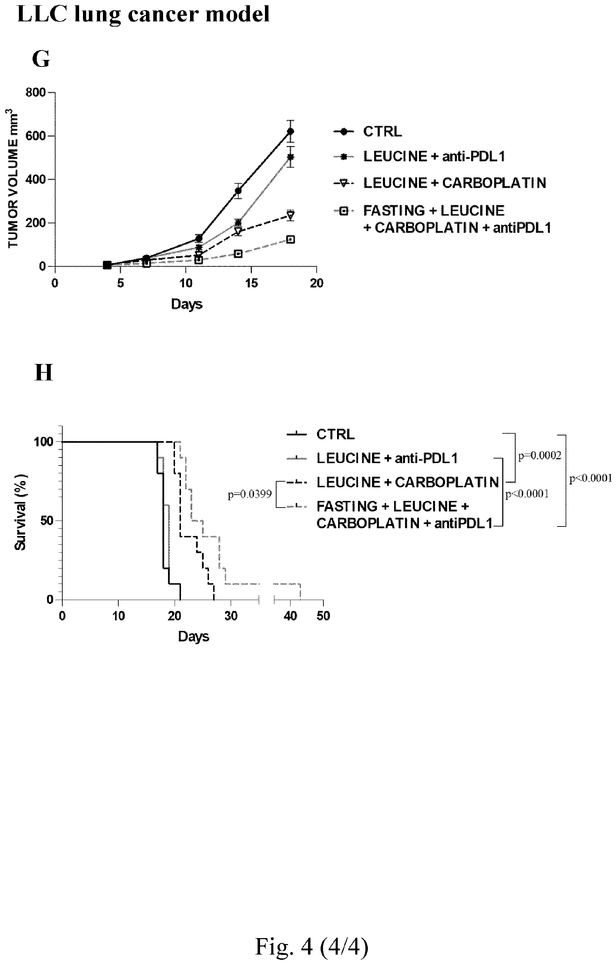

**FIG 4****. Impact of Leucine supplementation, alone or combined with platinum chemotherapy and anti-PD-L1 immunotherapy, on tumor growth and animal survival.**

A) Growth of 4T1-luc cell transplants in the mammary fat pad of 7-weeks old female BALB/c mice fed with standard diet or fasting cycles (48-hours of water-only fasting, followed by *ad libitum* refeeding, and repeated every 7 days), treated with Leucine (500 mg/kg), as administered once a day by oral gavage, plus/minus carboplatin (50 mg/kg), as administered once a week by intraperitoneal injection, plus/minus murine anti-PDL1 Monoclonal Antibody (MoAb) (100ug in each mouse) every-other-day by intraperitoneal injection, for a total number of 4 anti-PD-L1 treatment cycles (n=6-8). B) Survival curves of animals treated as described in A) are reported. C) Growth of E0771 cell transplants in the mammary fat pad of 7-weeks old female C57BL6/J mice fed with standard diet or fasting cycles (48-hours of water-only fasting, followed by *ad libitum* refeeding, and repeated every 7 days), treated with Leucine (500 mg/kg), as administered once a day by oral gavage, plus/minus paclitaxel (30 mg/kg), as administered once a week by intraperitoneal injection, plus/minus murine anti-PDL1 Monoclonal Antibody (MoAb) (100ug in each mouse) every-other-day by intraperitoneal injection, for a total number of 4 anti-PD-L1 treatment cycles (n=9-12). D) Survival curves of animals treated as described in C) are reported. E) Growth of CT26 cell transplants in the right flank of 7-weeks old female BALB/c mice fed with standard diet or fasting cycles (48-hours of water-only fasting, followed by *ad libitum* refeeding, and repeated every 7 days), treated with Leucine (500 mg/kg), as administered once a day by oral gavage, plus/minus oxaliplatin (5 mg/kg), as administered once a week by intraperitoneal injection, plus/minus murine anti-PDL1 Monoclonal Antibody (MoAb) (100ug in each mouse) every-other-day by intraperitoneal injection, for a total number of 4 anti-PD-L1 treatment cycles (n=6-7). F) Survival curves of animals treated as described in E) are reported. G) Growth of LLC lung cell transplants after cell injection in the right flank (subcutaneous tissue) of 7-weeks old female C57BL6/J fed with standard diet or fasting cycles (48-hours of water-only fasting, followed by *ad libitum* refeeding, and repeated every 7 days), treated with Leucine (500 mg/kg), as administered once a day by oral gavage, plus/minus carboplatin (50 mg/kg), as administered once a week by intraperitoneal injection, plus/minus murine anti-PDL1 Monoclonal Antibody (MoAb) (100ug in each mouse) every-other-day by intraperitoneal injection, for a total number of 4 anti-PD-L1 treatment cycles (n=10-15). H) Survival curves of mice treated as described in G) are reported.

Data are represented as mean ± SEM. P values were determined by ordinary one-way Anova or unpaired t-test for comparisons between only 2 groups.

Comparison of survival curves were performed with Log-rank (Mantel-Cox) test. P values ≤ 0.05 were considered significant.

### EXAMPLES

### Mouse models

Animals were housed under specific pathogen-free conditions with 12 hours day/light cycles. All experiments were performed in accordance with the guidelines established in the Principles of Laboratory Animal Care (directive 86/609/EEC), and they were approved by the Italian Ministry of Health. As for the syngeneic 4T1 TNBC model, 7-weeks old female BALB/c/Ola Hsd mice (Envigo) were injected in the mammary fat pad with 2×10⁴ 4T1-luc (luciferase-positive) cells resuspended in 20 µl of medium (RPMI supplemented with 10% FBS, 2mM glutamine). As for syngeneic E0771 TNBC model, 7-weeks old female C57BL6/J (Charles river) were injected in the mammary fat pad with 3×10⁵ E0771 cells resuspended in 20 µl of medium (RPMI supplemented with 10% FBS, 2mM glutamine). As for the syngeneic lung cancer model, 7-weeks old female C57BL6/J (Charles River) were injected subcutaneously with 2×10⁵LLC cells resuspended in 100 µl of PBS. Finally, for the establishment of the syngeneic colorectal cancer model, 7-weeks old female BALB/c/Ola Hsd mice (Envigo) were injected subcutaneously with 3×10⁵ CT26 cells resuspended in 100 µl of PBS. When tumors were palpable (7 days after cells inoculation for 4T1, E0771 and CT26 models, 5 days after cells inoculation for LLC model), mice were randomly assigned to different experimental groups. Tumor volumes were measured twice a week through a digital caliper according to the following equation: tumor volume (mm3) = length × width × thickness × 0,5. At the end of the experiments, mice were euthanized by using CO2.

### Animal diets and treatments

Mice were fed ad libitum with irradiated standard diet VRFI (P) diet (Charles River) containing 3,89 kcal/g of gross energy. According to the experimental groups, some mice were subjected to fasting one per week, consisting of 48-hour water-only-fasting, followed by 5 days of *ad libitum* refeeding (standard diet), and repeated until unacceptable animal toxicity or sacrifice. Mouse weight was monitored twice per week and during any fasting cycle. In the experiments, weight loss could not exceed 20% as compared to baseline values. Before initiating a new fasting cycle, the animals should have completely recovered their original bodyweight.

As for experiments employing the use of Leucine, mice were daily treated with 500 mg/kg of Leucine (Sigma Aldrich, # L8000), which was dissolved in physiologic water and administered by oral gavage.

As for the experiments employing anti-PDL1 immunotherapy, mice were treated with *InVivo*MAb anti-mouse PD-L1 (B7-H1) (BioXCell, # BE0101), at a dose of 100 ug per mouse, via intraperitoneal injection. Mice were treated with anti-PDL1 every other day, up to a maximum of 4 cycles (day 0, 2, 4, 6 after the beginning of the experiments).

As for experiments employing the use of chemotherapy, carboplatin (Sindan, 10mg/ml), oxaliplatin (Mylan, 5mg/ml in infusion solution) and paclitaxel (Accord, 6mg/ml in 527mg/ml polyoxyethylated castor oil and 391 mg/ml ethanol) were provided by Istituto Nazionale dei Tumori, Milano.

Carboplatin was administered at a dose of 50 mg/kg (final volume 100ul), once × week, via intraperitoneal injection; oxaliplatin was administered at a dose of 5 mg/kg (diluted in injectable solution, final volume 100ul), once per week, via intraperitoneal injection; paclitaxel was administered at a dose of 30 mg/kg (diluted in physiologic water, final volume 200ul), once × week, via intraperitoneal injection.

### Results

### Leucine supplementation retards tumor growth and synergizes with cyclic fasting in syngeneic TNBC, CRC and lung cancer models

The impact of oral Leucine supplementation, as given at a daily dose of 500 mg/Kg Leucine by oral gavage, was investigated on *in vivo* tumor growth and mouse survival in orthotopic (4T1 and E0771 TNBC cells injected in the mammary fat pad of female BALB/c mice and female C57BL6/J mice, respectively) or subcutaneous (CT26: murine colorectal carcinoma - CRC, or LLC: murine squamous lung carcinoma cells, injected in the subcutaneous tissue of female BALB/c mice and female C57BL6/J mice, respectively) syngeneic tumor models.

As shown in Figure 1, Leucine supplementation slowed down primary tumor growth and prolonged animal survival in all these models. As previously reported by other groups^{3, 4, 7} , cyclic fasting, consisting of 48-hour water-only fasting followed by *ad libitum* feeding, also slowed down tumor growth and prolonged animal survival. Of note, combining Leucine supplementation and cyclic fasting resulted in cooperative reduction of *in vivo* tumor growth, as well as in significant prolongation of animal survival (Figure 1).

Together, these results indicate that oral Leucine supplementation can enhance the antitumor activity of cyclic fasting/FMD, which has recently emerged as a promising antitumor strategy in several clinical settings^{6, 8, 9, 20, 21}.

### Leucine supplementation cooperates with anti-PD-L1 immunotherapy to retard tumor growth and to prolong animal survival in syngeneic mouse tumor models

Since immune checkpoint inhibitors, such as anti-PD1 or anti-PD-L1 Monoclonal Antibodies (MoAb), have recently emerged as effective antitumor treatments in patients with different malignancies, including early stage/advanced TNBC¹²⁻¹⁴, lung carcinomas¹⁵⁻¹⁸ and CRC¹⁹, the inventors investigated whether Leucine supplementation enhances the antitumor effects of anti-PD-L1 immunotherapy. For these experiments, syngeneic 4T1 (BALB/c mice), E0771 (C57BL/6 mice), CT26 (BALB/c) and LLC (C57BL/6 mice) cell transplants were used. As shown in Figure 2, anti-PD-L1 immunotherapy alone had different antitumor effects depending on the tumor model, with 4T1 and CT26 transplants being resistant to anti-PD-L1 MoAb, whereas E0771 mouse transplants were sensitive to anti-PD-L1 therapy, which also resulted in mild prolongation of animal survival when used as a single therapy. Notably, combining oral Leucine supplementation with anti-PD-L1 immunotherapy cooperatively slowed down *in vivo* tumor growth and prolonged animal survival; in addition, in some animals treated with this combination we observed completed disappearance of the primary tumor mass, with some mice also achieving long-term survival. Finally, in subcutaneous LLC model, which is also resistant to anti-PDL1 MoAb when used alone but it becomes sensitive when MoAb is combined with fasting⁴, combining Leu+fasting with anti-PD-L1 significantly slowed down tumor growth and prolonged animal survival when compared to anti-PDL1 MoAb + fasting.

Together, these results indicate that oral Leucine supplementation can sensitize immunotherapy-resistant tumors to the effects of anti-PD-L1 immunotherapy; at the same time, Leucine supplementation significantly enhances the antitumor effects of anti-PD-L1 MoAb in immunotherapy-sensitive malignancies. Of note, animals treated with the combination of oral Leucine and immunotherapy did not show any sign of suffering, thus suggesting that these combinations result in enhanced antitumor effects without causing severe adverse events.

Due to the widespread use of anti-PD1 or anti-PD-L1 immune checkpoint inhibitors in patients with both early stage and advanced TNBC, CRC or lung carcinomas, our findings indicate that oral Leucine supplementation is a promising therapeutic strategy to boost the antitumor activity and efficacy of anti-PD1 or anti-PD-L1 MoAb regardless of primary tumor sensitivity/resistance to immunotherapy.

### Leucine supplementation enhances antitumor activity and efficacy of chemotherapy in TNBC, CRC and lung cancers models

Despite the recent advent of new effective treatments, such as immunotherapy or targeted therapies, cytotoxic chemotherapy, including platinum (i.e., cisplatin, carboplatin or oxaliplatin) or taxane (paclitaxel, docetaxel, nab-paclitaxel)-based chemotherapy remains the most effective, standard-of-care treatment option for patients with early-stage or advanced malignancies, including TNBC and CRC.

Here, we investigated if oral Leucine supplementation cooperates with platinum/taxane chemotherapy in syngeneic models of TNBC (orthotopic 4T1 or E0771 transplants in female BALB/c and C57BL6/J mice, respectively) and in subcutaneous models of CRC and lung cancer. As shown in Figure 3, weekly intraperitoneal (i.p.) injection of carboplatin (4T1 and LLC), paclitaxel (E0771) or oxaliplatin (CT26) slowed down *in vivo* tumor growth and resulted in statistically significant prolongation of animal survival, with the therapeutic effect being different according to the tumor type (i.e., more evident in E0771 transplants, and less evident in 4T1, LLC and CT26 transplants).

Of note, oral Leucine supplementation cooperated with chemotherapy in slowing down *in vivo* tumor growth and in prolonging animal survival. Again, the most relevant effects of combination treatment (Leu plus chemotherapy), in terms of reduced primary tumor growth progression and animal survival prolongation, was observed in mouse E0771 transplants, while it was less evident, yet still statistically significant, in 4T1, LLC and CT26 mouse transplants.

Together, our results indicate that oral Leucine supplementation enhances the antitumor activity and efficacy of platinum- andtaxane-based chemotherapy in highly aggressive and rapidly deadly mouse models of TNBC and CRC.

### Combining oral Leucine supplementation with chemo-immunotherapy and fasting results in long-term survival and in some animal cures in highly aggressive TNBC and CRC mouse models

The goal of the current preclinical and clinical research in Oncology is to prolong the survival of patients with highly aggressive malignancies and, eventually, to cure some of these patients. However, this is a difficult goal to achieve, mainly as a result of tumor heterogeneity and to the difficulty to eradicate all tumor cells.

Here, we investigated whether combining chemo-immunotherapy (namely, platinum- or taxane-based chemotherapy plus anti-PD-L1 MoAb), which is now the standard-of-care treatment in several patients with advanced malignancies, including advanced TNBC and lung carcinoma patients, with oral Leucine plus cyclic fasting (an experimental antitumor intervention that showed promising antitumor effects - see Figure 1), can result in long-term animal survival.

For these experiments, we used the same TNBC (4T1, E0071) and CRC (CT26) and lung cancer (LLC) mouse models used in previous experiments. As shown in Figure 4, combining chemo-immunotherapy with oral Leucine (daily 500 mg/kg Leucine by oral gavage) plus cyclic fasting (48-hour water-only fasting followed by *ad libitum* refeeding, and repeated every 7 days) results in meaningful prolongation of animal survival when compared to the fasting-Leucine doublet. In addition, and even more importantly in terms of potential translational implications, the quadruple treatments resulted in complete regression of the primary tumor in some mice bearing E0771 or CT26 cell transplants, and some animals were still alive some months after the initiation of these treatments.

Together, these data suggest that the combination of an experimental therapy, such as oral Leucine supplementation plus cyclic fasting/FMD, with standard chemoimmunotherapy combinations can result in long-term animal survival and animal cure in highly aggressive and deadly models of TNBC, CRC and lung cancer.

### REFERENCES

1 Miller, K. D. et al. Cancer treatment and survivorship statistics, 2022. CA Cancer J Clin 72, 409-436, doi:10.3322/caac.21731 (2022).
2 Di Biase, S. et al. Fasting-Mimicking Diet Reduces HO-1 to Promote T Cell-Mediated Tumor Cytotoxicity. Cancer Cell 30, 136-146, doi:10.1016/j.ccell.2016.06.005 (2016).
3 Di Tano, M. et al. Synergistic effect of fasting-mimicking diet and vitamin C against KRAS mutated cancers. Nat Commun 11, 2332, doi:10.1038/s41467-020-16243-3 (2020).
4 Ajona, D. et al. Short-term starvation reduces IGF-1 levels to sensitize lung tumors to PD-1 immune checkpoint blockade. Nature Cancer 1, 75-85, doi:10.1038/s43018-019-0007-9 (2020).
5 Cortellino, S. et al. Fasting renders immunotherapy effective against low-immunogenic breast cancer while reducing side effects. Cell Rep 40, 111256, doi:10.1016/j.celrep.2022.111256 (2022).
6 Caffa, I. et al. Fasting-mimicking diet and hormone therapy induce breast cancer regression. Nature 583, 620-624, doi:10.1038/s41586-020-2502-7 (2020).
7 Lee, C. et al. Fasting cycles retard growth of tumors and sensitize a range of cancer cell types to chemotherapy. Sci Transl Med 4, 124ra127, doi:10.1126/scitranslmed.3003293 (2012).
8 Vernieri, C. et al. Fasting-Mimicking Diet Is Safe and Reshapes Metabolism and Antitumor Immunity in Patients with Cancer. Cancer Discov 12, 90-107, doi:10.1158/2159-8290.CD-21-0030 (2022).
9 Ligorio, F. et al. Exceptional tumour responses to fasting-mimicking diet combined with standard anticancer therapies: A sub-analysis of the NCT03340935 trial. Eur J Cancer 172, 300-310 doi:10.1016/j.ejca.2022.05.046 (2022).
10 Delgoffe, G. M. et al. The kinase mTOR regulates the differentiation of helper T cells through the selective activation of signaling by mTORC1 and mTORC2. Nat Immunol 12, 295-303, doi:10.1038/ni.2005 (2011).
11 Sinclair, L. V. et al. Control of amino-acid transport by antigen receptors coordinates the metabolic reprogramming essential for T cell differentiation. Nat Immunol 14, 500-508, doi:10.1038/ni.2556 (2013).
12 Schmid, P. et al. Pembrolizumab for Early Triple-Negative Breast Cancer. N Engl J Med 382, 810-821, doi:10.1056/NEJMoa1910549 (2020).
13 Cortes, J. et al. Pembrolizumab plus Chemotherapy in Advanced Triple-Negative Breast Cancer. N Engl J Med 387, 217-226, doi:10.1056/NEJMoa2202809 (2022).
14 Schmid, P. et al. Atezolizumab and Nab-Paclitaxel in Advanced Triple-Negative Breast Cancer. N Engl J Med 379, 2108-2121, doi:10.1056/NEJMoa1809615 (2018).
15 Gandhi, L. et al. Pembrolizumab plus Chemotherapy in Metastatic Non-Small-Cell Lung Cancer. N Engl J Med 378, 2078-2092, doi:10.1056/NEJMoa1801005 (2018).
16 Chung, H. C. et al. Pembrolizumab After Two or More Lines of Previous Therapy in Patients With Recurrent or Metastatic SCLC: Results From the KEYNOTE-028 and KEYNOTE-158 Studies. J Thorac Oncol 15, 618-627, doi:10.1016/j.jtho.2019.12.109 (2020).
17 Herbst, R. S. et al. Atezolizumab for First-Line Treatment of PD-L1-Selected Patients with NSCLC. N Engl J Med 383, 1328-1339, doi:10.1056/NEJMoa1917346 (2020).
18 Horn, L. et al. First-Line Atezolizumab plus Chemotherapy in Extensive-Stage Small-Cell Lung Cancer. N Engl J Med 379, 2220-2229, doi:10.1056/NF-JMoa1809064 (2018).
19 Andre, T. et al. Pembrolizumab in Microsatellite-Instability-High Advanced Colorectal Cancer. N Engl J Med 383, 2207-2218, doi:10.1056/NEJMoa2017699 (2020).
20 Valdemarin, F. et al. Safety and Feasibility of Fasting-Mimicking Diet and Effects on Nutritional Status and Circulating Metabolic and Inflammatory Factors in Cancer Patients Undergoing Active Treatment. Cancers (Basel) 2021 Aug 9;13(16):4013. doi: 0.3390/cancers13164013.
21 de Groot, S. et al. Fasting mimicking diet as an adjunct to neoadjuvant chemotherapy for breast cancer in the multicentre randomized phase 2 DIRECT trial. Nat Commun. 2020 Jun 23;11(1):3083. doi: 10.1038/s41467-020-16138-3. PMID: 32576828; PMCID: PMC7311547.
22 Nagamori, S. et al. Structure-activity relations of leucine derivatives reveal critical moieties for cellular uptake and activation of mTORC1-mediated signaling. Ammino Acid 2016; 48: 1045-1058. doi: 10.1007/s00726-015-2158-z

## Claims

1. Leucine or a pharmaceutically acceptable salt thereof for use in the treatment of cancer either alone or in combination with at least one fasting cycle or with a fasting mimicking diet and/or a further therapeutic intervention.

2. Leucine or the pharmaceutically acceptable salt thereof for use in the treatment of cancer according to claim 1 wherein said at least one fasting cycle is **characterized by** 24 h to 72 h, preferably 24 h to 48 h, preferably 48 h of water-only 100% caloric restriction.

3. Leucine or the pharmaceutically acceptable salt thereof for use according to claim 1, wherein said fasting mimicking diet lasts for a period between 24 and 190 hours, preferably said fasting mimicking diet lasts for a period of 24 to 150 hours, preferably said fasting mimicking diet lasts for approximately 120 hours.

4. Leucine or the pharmaceutically acceptable salt thereof for use according to claim 3, wherein said fasting mimicking diet is a regular caloric intake reduced by 65% to 85% and/or said fasting mimicking diet is based on an average caloric intake of between 200 and 1200 Kcal/day, preferably between 300 and 600 Kcal/day600 Kcal/day.

5. Leucine or the pharmaceutically acceptable salt thereof for use according to claim 4, wherein said fasting mimicking diet comprises a first period of 0 to 24 hours wherein the caloric intake is a regular caloric intake reduced by approximately 65%, followed by a second period of 24 to 96 hours wherein the caloric intake is a regular caloric intake reduced by approximately 85%, preferably said first period lasts approximately 24 hours and said second period lasts approximately from 48 to 96 hours.

6. Leucine or the pharmaceutically acceptable salt thereof for use according to any one of claims 3 to 5, wherein said fasting mimicking diet comprises a reduced protein intake and/or a reduced simple carbohydrate (sugars) intake.

7. Leucine or the pharmaceutically acceptable salt thereof for use according to anyone of claims 3 to 6, wherein said fasting mimicking diet comprises administering a food having a content of monounsaturated and/or polyunsaturated fats between 10% and 80 % of the total calorie amount, a content of proteins from 0 to 10% of the total calorie amount, and a content of carbohydrates from 5% to 50% of the total calorie amount.

8. Leucine or the pharmaceutically acceptable salt thereof for use according to any one of previous claims, wherein said leucine is administered in an amount of approximately from 100mg/kg and 1000mg/kg, preferably between 150 mg/kg and 800mg/kg, preferably between 250 mg/kg and 600 mg/kg.

9. Leucine or the pharmaceutically acceptable salt thereof for use according to any one of previous claims, wherein said further therapeutic intervention is selected from the group consisting of: surgery, radiotherapy and a further therapeutic agent.

10. Leucine or the pharmaceutically acceptable salt thereof for use according to claim 8, wherein said further therapeutic agent is an immune checkpoint inhibitor (ICI), preferably a PD-1 inhibitor, and/or PDL-1 inhibitor and/or CTLA-4 inhibitor.

11. Leucine or the pharmaceutically acceptable salt thereof for use according to claim 9, wherein said further therapeutic agent is a chemotherapeutic agent, preferably said chemotherapeutic agent is selected from the group consisting of: a DNA synthesis inhibitor, a DNA damaging agent, a topoisomerase inhibitor, a microtubule poison, preferably said chemotherapeutic agent is selected from the group consisting of: oxaliplatin, carboplatin, cisplatin, paclitaxel, docetaxel, nab-paclitaxel, eribulin, doxorubicin, epirubicin, irinotecan, gemcitabine, cyclophosphamide.

12. Leucine or the pharmaceutically acceptable salt thereof or the leucine enriched polypeptide for use according to claim 9, wherein said further therapeutic agent is a combination of chemotherapeutic agent and an immunotherapy agent, preferably said chemotherapeutic agent is selected from the group consisting of: a DNA synthesis inhibitor, a DNA damaging agent, a topoisomerase inhibitor, a microtubule poison, preferably said chemotherapeutic agent is selected from the group consisting of: oxaliplatin, carboplatin, cisplatin, paclitaxel, docetaxel, nab-paclitaxel, eribulin, doxorubicin, epirubicin, irinotecan, gemcitabine, cyclophosphamide; and the immunotherapy agent is an immune checkpoint inhibitor (ICI), preferably a PD-1 inhibitor, and/or PDL-1 inhibitor and/or CTLA-4 inhibitor.

13. Leucine or the pharmaceutically acceptable salt thereof for use according to any one of previous claims, wherein said cancer is selected from the group consisting of: breast cancer, colorectal cancer, small cell and non small cell lung carcinomas (SCLCs and NSCLCs), pancreatic adenocarcinoma, colon cancer, rectal cancer, mucinous adenocarcinoma, melanoma, biliary tract carcinoma, gastric carcinoma, esophageal carcinoma, small intestine carcinoma, neuroblastomas, gliomas, sarcomas, lymphomas, leukemias, neuroendocrine carcinomas, either early stage or metastatic.

14. Leucine or the pharmaceutically acceptable salt thereof for use according to any one of previous claims, wherein said cancer is resistant to conventional therapy, preferably said cancer is resistant to radiotherapy or chemotherapy, preferably said cancer is resistant to: a DNA synthesis inhibitor, a DNA damaging agent, a topoisomerase inhibitor, a microtubule poison, preferably said chemotherapeutic agent is selected from the group consisting of: oxaliplatin, carboplatin, cisplatin, paclitaxel, docetaxel, nab-paclitaxel, eribulin, doxorubicin, epirubicin, irinotecan, gemcitabine, cyclopshosphamide.

15. A pharmaceutical or nutritional composition or dietary supplement comprising leucine or the pharmaceutically acceptable salt thereof for use according to any one of previous claims, and a pharmaceutical or nutritionally acceptable carrier.

16. The pharmaceutical or nutritional composition or dietary supplement for use according to claim 15 formulated as an oral formulation with delayed or prolonged intestinal absorption.
